(19) Europäisches Patentamt / European Patent Office / Office européen des brevets

(11) **EP 1 765 752 B1**

(12) **EUROPÄISCHE PATENTSCHRIFT**

(45) Veröffentlichungstag und Bekanntmachung des Hinweises auf die Patenterteilung:
**26.08.2015  Patentblatt 2015/35**

(21) Anmeldenummer: **05756999.8**

(22) Anmeldetag: **18.06.2005**

(51) Int Cl.:
*C07C 37/68* *(2006.01)*     *C07C 37/86* *(2006.01)*
*C07C 37/74* *(2006.01)*

(86) Internationale Anmeldenummer:
**PCT/EP2005/006603**

(87) Internationale Veröffentlichungsnummer:
**WO 2006/002788 (12.01.2006 Gazette 2006/02)**

(54) **VERFAHREN ZUR ABTRENNUNG VON PHENOL AUS PHENOLHALTIGEN STRÖMEN AUS DER HERSTELLUNG VON BISPHENOL A**

METHOD FOR SEPARATING PHENOL FROM STREAMS CONTAINING PHENOL THAT HAVE BEEN OBTAINED DURING THE PRODUCTION OF BISPHENOL A

PROCEDE POUR SEPARER DU PHENOL DE COURANTS CONTENANT DU PHENOL ISSUS DE LA PRODUCTION DE BISPHENOL A

(84) Benannte Vertragsstaaten:
**BE DE ES NL**

(30) Priorität: **02.07.2004  DE 102004032232**

(43) Veröffentlichungstag der Anmeldung:
**28.03.2007  Patentblatt 2007/13**

(73) Patentinhaber: **Bayer Intellectual Property GmbH
40789 Monheim (DE)**

(72) Erfinder:
• **PREIN, Michael
  47809 Krefeld (DE)**
• **EEK, Rob c/o Bayer Material Science Limited
  76/F The Center,
  Hong Kong (HK)**
• **AUDENAERT, Raymond
  B-9920 Hamme (BE)**

(74) Vertreter: **BIP Patents
c/o Bayer Intellectual Property GmbH
Alfred-Nobel-Straße 10
40789 Monheim am Rhein (DE)**

(56) Entgegenhaltungen:
**EP-A- 0 758 637     EP-A- 0 812 815**

## Beschreibung

Bisphenole als Kondensationsprodukte von Phenolen und Carbonylverbindungen sind Ausgangsstoffe oder Zwischenprodukte zur Herstellung einer Vielzahl kommerzieller Produkte. Von besonderer technischer Bedeutung ist das Kondensationsprodukt aus der Reaktion zwischen Phenol und Aceton, 2,2-Bis(4-hydroxyphenyl)propan (Bisphenol A, BPA). BPA dient als Ausgangstoff zur Herstellung verschiedenartiger polymerer Werkstoffe wie beispielsweise Polyarylate, Polyetherimide, Polysulfone und modifizierter Phenol-Formaldehydharze. Bevorzugte Anwendungsgebiete liegen in der Herstellung von Epoxyharzen und Polycarbonaten.

Die technisch relevanten Herstellmethoden für BPA beruhen auf der säurekatalysierten Umsetzung von Phenol mit Aceton, wobei bevorzugt ein Phenol-Aceton-Verhältnis von > 5 : 1 in der Reaktion eingestellt wird. Als saure Katalysatoren können homogene wie auch heterogene Brönsted- oder Lewissäuren genutzt werden, so beispielsweise starke Mineralsäuren wie Salz- oder Schwefelsäure. Bevorzugt kommen gelförmige oder makroporöse sulfonierte vernetzte Polystyrolharze (saure Ionentauscher) zum Einsatz. Die nachfolgenden Ausführungen beziehen sich auf ein Verfahren unter Nutzung von sauren Ionentauschern als Katalysatoren. Diese können mono- oder heterodispers sein.

Zur Erzielung hoher Selektivitäten wird die Umsetzung von Phenol mit Aceton in Gegenwart geeigneter Mercaptoverbindungen als Cokatalysatoren durchgeführt. Diese können entweder homogen in der Reaktionslösung gelöst sein oder über ionische oder kovalente Bindungen an der sulfonierten Poylstyrolmatrix fixiert werden. Die Reaktionseinheit ist ein Schichtbett oder Wirbelbett, die auf- oder abwärts durchflossen werden, oder eine Kolonne nach Art einer Reaktivdestillationskolonne.

Die Selektivität der Umsetzung wie auch die Langzeitstabilität des Katalysators wird in maßgeblicher Weise von der Qualität der eingesetzten Rohstoffe Phenol und Aceton beeinflusst. Insbesondere zur Herstellung von BPA als Rohstoff für hochwertige Kunststoffe wie beispielsweise Polycarbonat werden daher sehr hohe Reinheitsanforderungen an die eingesetzten Grundstoffe Phenol und Aceton gestellt. Typischerweise werden hierbei Reinheiten von > 99,95 Gew.-% für Phenol und > 99,90 Gew.-% für Aceton bei gleichzeitig niedrigen Fremdstoffgehalten (S < 0,5 ppm, Fe < 1 ppm) als positiv zur Erzielung hoher Produktreinheiten und zur Minimierung von Katalysatordeaktivierung angesehen. So wird in EP-A-876 319 beschrieben, dass kommerzielles Phenol durch Behandlung mit Molekularsieb von störenden Verunreinigungen befreit wird und dadurch eine bessere Verwendbarkeit in einem Prozess zur BPA-Herstellung gewährleistet

## Angepasste Beschreibung

wird. EP-A-680 913 beschreibt die Verwendung von modifizierten sauren Ionentauschern zur Entfernung von Hydroxyaceton aus Phenol zur BPA-Synthese.

EP 812 815 A2 beschreibt ein integriertes Verfahren zur Herstellung von Bisphenol, wobei Phenol und Keton in Anwesenheit eines Ionenaustauschers als Katalysator zu einem Gemisch reagiert, das ein Bisphenol enthält. EP 812 815 A2 beschreibt allerdings keine Aufarbeitung von Restharz, in dem noch größere Mengen Phenol enthalten sind, der durch Entsorgung zu einem Verlust an Rohstoffen führt.

Bei der Umsetzung von Phenol mit Aceton in Gegenwart saurer Katalysatoren und Mercaptoverbindungen als Cokatalysatoren entsteht eine Produktmischung, die neben nicht umgesetztem Phenol und gegebenenfalls Aceton in erster Linie BPA und Wasser enthält. Daneben treten in geringen Mengen typische Nebenprodukte der Kondensationreaktion auf, so beispielsweise 2-(4-hydroxyphenyl)-2-(2-hydroxyphenyl)propan (o,p-BPA), substituierte Indane, Hydroxyphenylindanole, Hydroxyphenyl-chromane, substituierte Xanthene und höher kondensierte Verbindungen mit drei oder mehr Phenylringen im Molekülgerüst.

Die genannten Nebenprodukte wie auch Wasser, Phenol und Aceton beeinträchtigen die Eignung von BPA zur Herstellung von Polymeren und müssen durch geeignete Verfahren abgetrennt werden. Insbesondere zur Herstellung von Polycarbonat werden hohe Reinheitsanforderungen an den Rohstoff BPA gestellt.

Die Aufarbeitung und Reinigung von BPA erfolgt durch eine mehrstufige Kaskade von geeigneten Reinigungsverfahren wie beispielhaft Suspensionskristallisation, Schmelzekristallisation, Destillation und/oder Desorption. In einer technisch bevorzugten Ausführungsform erfolgt die Abtrennung von BPA aus der Reaktionsmischung in Form eines etwa äquimolaren kristallinen Addukts mit Phenol durch Abkühlen der Reaktionsmischung unter Auskristallisieren des BPA/Phenol-Addukts. Die Kristallisation erfolgt bevorzugt als Suspensionskristallisation. Die BPA/Phenol-Adduktkristalle werden anschließend durch eine geeignete Apparatur zur Fest-Flüssigtrennung wie Drehfilter oder Zentrifugen von der Flüssigphase abgetrennt und der weiteren Reinigung zugeführt. So erhaltene Adduktkristalle weisen typischerweise ein Reinheit von > 99 Gew.-% BPA bezogen auf BPA und die Nebenkomponenten bei einem Phenolanteil von ca. 40 Gew.-% bezogen auf die gesamte Adduktkristallmenge auf. Durch Waschen mit geeigneten Lösungen, die typischerweise eine oder mehrere Komponenten aus der Gruppe Aceton, Wasser, Phenol, BPA und Nebenkomponenten enthalten, können die Adduktkristalle von oberflächlich anhaftenden Verunreinigungen befreit werden. Die im Anschluss an die oben beschriebene Suspensionskristallisation der Reaktionslösung und Fest-Flüssig-Trennung erhaltenen BPA-Phenol-Adduktkristalle werden weitergehenden Reinigungsschritten zugeführt, wobei die Abtrennung von Phenol durchgeführt wird und gegebenenfalls durch den Einsatz geeigneter Reinigungsoperationen (Suspensionskristallisation, Schichtkristallisation, Extraktion, Destillation) eine Verringerung der Konzentration an Nebenkomponenten erzielt wird.

Der bei der Fest-Flüssigtrennung anfallende Flüssigstrom (Mutterlauge) enthält Phenol, BPA, bei der Reaktion entstandenes Wasser, nicht umgesetztes Aceton und ist angereichert an den bei der BPA-Herstellung typischerweise anfallenden Nebenkomponenten. Dieser Mutterlaugenstrom wird in einer bevorzugten Ausführungsform in die Reaktionseinheit zurückgeführt. Um die katalytische Aktivität der sauren Ionentauscher aufrecht zu erhalten wird zuvor entstandenes Wasser ganz oder teilweise durch Destillation entfernt, wobei auch gegebenenfalls noch vorhandenes Aceton ganz oder teilweise aus der Mutterlauge entfernt wird. Der so erhaltene entwässerte Reaktionsstrom wird um Phenol und Aceton ergänzt und in die Reaktionseinheit zurückgeführt. Alternativ können auch vor Durchführung der Suspensionskristallisation des BPA-Phenol-Addukts Wasser und Aceton ganz oder teilweise destillativ entfernt werden. Bei den genannten Destillationsschritten kann auch eine Teilmenge des in der Reaktionslösung vorhandenen Phenols destillativ abgetrennt werden.

Bei einer derartigen Kreislauffahrweise tritt als Problem auf, dass Nebenprodukte der BPA-Herstellung im Kreislaufstrom angereichert werden, die die Reinheit von BPA in der Suspensionkristallisation negativ beeinflussen und zur Desaktivierung des Katalysatorsystems führen können. Um eine übermäßige Anreicherung von Nebenkomponenten im Kreislaufstrom zu vermeiden, muss eine Teilmenge des Mutterlaugengemischs aus dem System ausgespeist werden. Die auf diesem Wege aus dem Prozess entnommene Menge an Nebenkomponenten muss im Gleichgewichtszustand der in der Reaktion gebildeten Menge an Nebenkomponenten entsprechen. Diese Ausspeisung erfolgt typischerweise derart, dass eine Teilmenge der Mutterlauge aus dem Kreislaufstrom entnommen wird, wobei zuvor optional gebildetes Reaktionswasser, nicht umgesetztes Aceton und Teilmengen Phenol destillativ entfernt werden können. Die Zusammensetzung der Mutterlauge an dieser Stelle und somit auch die Zusammensetzung der ausgespeisten Teilmenge besteht typischerweise aus 70 bis 90 Gew.-% Phenol, 3 bis 15 Gew.-% BPA und 3 bis 15 Gew.-% Nebenkomponenten und Isomeren, die in der Reaktion gebildet wurden. Da letztlich lediglich der letztgenannte Anteil an Nebenkomponenten aus dem Prozess entfernt werden muss, wird die ausgespeiste Menge weiteren Aufarbeitungsschritten unterworfen, um Materialverluste zu minimieren.

In einer einfachen Ausführungsform wird hierbei Phenol bis auf einen Restgehalt von <10 Gew.-% abdestilliert, so dass ein Restharz mit einem Gehalt von < 10 Gew.-% Phenol, 15 bis 85 Gew.-% BPA und 15 bis 85 Gew.-% Nebenkomponenten erhalten wird, das aus dem Prozess entnommen und beispielsweise durch Verbrennung oder Deponierung entsorgt wird.

In einer anderen Ausführungsform wird ein Teil des in der ausgespeisten Menge enthaltenen BPA zurückgewonnen, indem man aus dem ausgespeisten Teilstrom einen Teil des Phenol abdestilliert und die so erhaltenen angereicherte Lösung einer Suspensionkristallisation und anschließenden Fest-Flüssig-Trennung zuführt. Hierbei hat es sich als vorteilhaft erwiesen, die ausgespeiste Menge vor oder nach der teilweisen Abtrennung von Phenol über eine mit saurem Ionentauscher befüllte Umlagerungseinheit zu führen. Diese Einheit wird im allgemeinen bei höheren Temperaturen betrieben als die Reaktionseinheit. In dieser Umlagerungseinheit werden unter den vorherrschenden Bedingungen einige der im Kreislaufstrom vorhandenen Nebenkomponenten der BPA-Herstellung zu BPA isomerisiert, so dass die Gesamtausbeute an BPA erhöht werden kann. In der Fest-Flüssig-Trennung wird ein Teil des enthaltenen BPA als BPA-Phenol-Adduktkristall erhalten und kann weiteren Reinigungsschritten zugeführt werden. Daneben wird ein Filtrat bestehend aus typischerweise 60 bis 90 Gew.-% Phenol, 3 bis 12 Gew.-% BPA und 3 bis 18 Gew.-% Nebenkomponenten erhalten. Aus diesem Filtrat wird das enthaltenen Phenol typischerweise bis auf < 10 Gew.-% Restgehalt abdestilliert und das erhaltenen Restharz enthaltend < 10 Gew.-% Phenol, 14 bis 80 Gew.-% BPA, 20 bis 86 Gew.-% Nebenkomponenten der Entsorgung zugeführt.

Den beschriebenen Prozessen zur Aufarbeitung des Ausspeisestroms haftet der Nachteil an, dass im letztlich entsorgten Restharz noch größere Mengen Phenol entweder als Substanz oder gebunden in BPA oder den Nebenkomponenten anwesend ist. Die Entsorgung des Restharzes führt somit zu einem Verlust an Rohstoffen.

Die Aufgabe der vorliegenden Erfindung bestand daher darin, ein Verfahren zur Aufarbeitung von Ausspeisströmen aus einem BPA-Produktionsprozess zu finden, mit dem Phenol in hoher Reinheit und mit hohen Ausbeuten erhalten wird.

Ein solches Verfahren erfüllt bevorzugt die folgenden Anforderungen:

1. Minimierung von Phenol im Restharz,

2. Minimierung von BPA im Restharz,

3. Minimierung der Restharzmenge,

4. Bereitstellung von Phenol hoher Reinheit (> 99,8 %) und geringen Anteilen an Verunreinigungen (S, Fe, Cl) aus dem Aufarbeitungsprozess mit hohen Ausbeuten,

5. Kontinuierliche Prozessführung bei gleichzeitigem minimierten Einsatz von Apparaten und Energie

Die Erfindung betrifft ein Verfahren zur kontinuierlichen Abtrennung von Phenol aus einem bei der Herstellung von Bisphenol A erzeugten Teilstrom enthaltend 40 bis 90 Gew.-% Phenol, 5 bis 40 Gew.-% Bisphenol A sowie 5 bis 40 Gew.-% Nebenkomponenten, die bei der Umsetzung von Phenol und Aceton zu Bisphenol A anfallen, bei dem man

a) den Teilstrom einer Destillationskolonne enthaltend mindestens 5 theoretische Trennstufen zuführt, und

b) in der Destillationskolonne Phenol über Kopf abdestilliert, und

c) einen ersten Teil des Sumpfprodukts aus dem Prozess ausschleust, und

d) einen zweiten Teil des Sumpfprodukts kontinuierlich in einen Verweilzeitbehälter überführt, in dem man die in dem Sumpfprodukt enthaltenen Nebenkomponenten bei Temperaturen von > 190°C und einer hydrodynamischen Verweilzwit von mindestens 120 min in Gegenwart eines sauren Katalysators zumindest teilweise isomerisiert, und anschließend zurück in die Destillationskolonne führt, wobei der Massenstrom des in den Verweilzeitbehälter geführten Teil des Sumpfprodukts mehr als 30% des Massenstroms des in Schritt a) in die Destillationskolonne eingebrachten Teilstroms beträgt.

Das in Schritt b) abdestillierte Phenol weist bevorzugt eine Reinheit von > 99,8 Gew.-% auf.

Die technische Aufgabe wird durch säurekatalysierte Spaltung des Ausspeisestroms in einem Verweilzeitbehälter und kontinuierliche Abtrennung des Phenols über eine Vakuum-Kolonne mit hohem Trennvermögen gelöst.

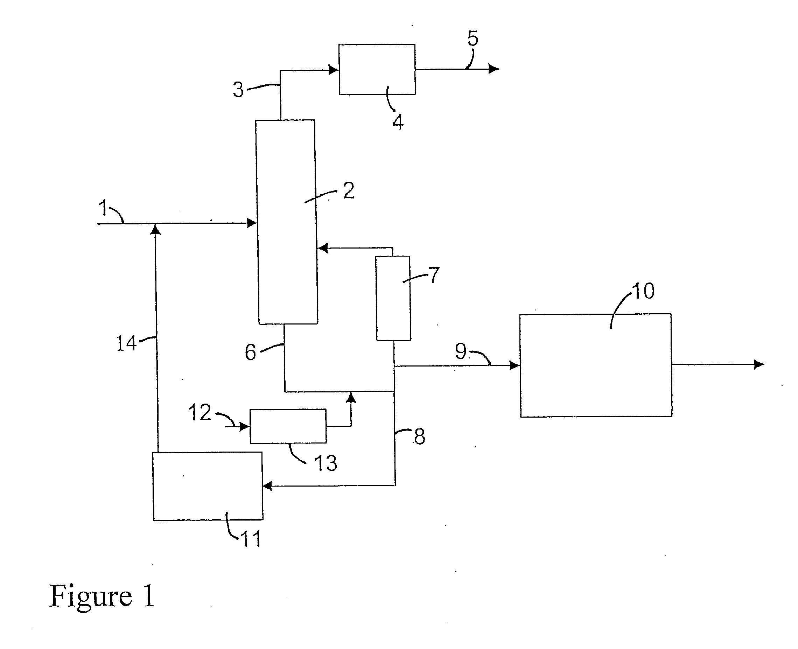

Figur 1 zeigt eine bevorzugte Ausführungsform des erfindungsgemäßen Verfahrens.

Hierbei wird ein aus einem Prozess zur Herstellung von Bisphenol A ausgespeister Teilstrom 1 in eine Destillationskolonne 2 mit hoher Trennleistung eingespeist. Die hohe Trennleistung ergibt sich dabei durch die mindestens 5 theoretischen Trennstufen. In dieser Destillationskolonne werden über Kopf Phenoldämpfe 3 abgetrennt, die in einem Kühler 4 zu flüssigem Phenol 5 kondensiert werden. Das Sumpfprodukt bzw. der Bodenaustrag 6 der Kolonne 2 wird über einen Wärmetauscher 7 im Kreis geführt, so dass auf diesem Wege die zur Verdampfung von Phenol benötigte Energie in das System eingetragen wird. Der Kühler 4 und der Verdampfer 7 können unabhängige Installationsteile sein oder auch baulich in die Destillationskolonnen integriert sein. Ein Teilstrom 9 des Sumpfprodukts wird gegebenenfalls nach Lagerung in einem Puffertank 10 als Restharz der Entsorgung zugeführt. Ein anderer Teilstrom 8 des Sumpfprodukts wird einem Verweilzeittank 11 zugeführt. In diesem Verweilzeittank erfolgt die säurekatalysierte Isomerisierung und Spaltung von BPA und Nebenkomponenten unter Bildung von Phenol. Die Spaltung wird durch die Zugabe geeigneter Säuren (Strom 12) durch eine Dosiereinheit 13 beschleunigt. Der phenolhaltende Ablauf 14 des Verweilzeitbehälters 11 wird mit dem ausgespeisten Teilstrom 1 vereinigt und so in die Destillationseinheit 2 zur Rückgewinnung von Phenol zurückgeführt.

Der Prozess wird kontinuierlich betrieben und erreicht nach kurzer Zeit einen Gleichgewichtszustand. Der aus dem Prozess zur Herstellung von Bisphenol A ausgespeiste Teilstrom 1 kann prinzipiell jeder Produktstrom aus einem Prozess zur Herstellung von BPA sein, der folgende Komponenten erhält: 40 bis 90 Gew.-% Phenol, 5 bis 40 Gew.-% Bisphenol A sowie 5 bis 40 Gew.-% Nebenkomponenten, die bei der Umsetzung von Phenol und Aceton zu Bisphenol A anfallen:

Bevorzugt ist Teilstrom 1 ein ausgespeister Teilstrom aus einem BPA-Herstellprozess mit Suspensionskristallisation und Fest-Flüssigtrennung, wobei ein Teilstrom des Filtrats der Fest-Flüssigtrennung zur Ausspeisung von Nebenprodukten in Richtung des erfindungsgemäßen Verfahrens zur Abtrennung von Phenol abgeführt wird. Optional wird hierbei der genannte Teilstrom 1 vor der Einspeisung in die Destillationskolonne 2 durch eine zusätzliche Umlagerungseinheit geführt, wobei durch Behandeln mit saurem Ionentauscher, anschließendes partielles Abdestillieren von Phenol, Kristallisation und Fest-Flüssigtrennung ein Teil des im Filtrat enthaltenen BPA wiedergewonnen und dem Hauptprozess zugeführt wird. In diesem Fall dient das Filtrat der Fest-Flüssigtrennung der Umlagerungseinheit als Einspeisestrom 1 der Destillationskolonne 2.

Um eine möglichst effiziente Spaltung, Abtrennung und Aufreinigung der phenolhaltigen Reststoffe zu ermöglichen, müssen beim erfindungsgemäßen Vorgehen folgende Randbedingungen beachtet werden: die Destillationskolonne 2 muss über mindestens 5 theoretische Trennstufen, bevorzugt mindestens 10 theoretische Trennstufe verfügen, um die Abtrennung anderer leichtsiedender Komponenten wie beispielsweise Isopropenylphenol zu ermöglichen und eine Aufreinigung des entstehende Phenols auf eine Reinheit von > 99,8 Gew.-% am Kopf der Kolonne zu gewährleisten. Bevorzugt wird die Destillation dabei mit einem absoluten Druck am Kopf der Kolonne von 70 bis 200 mbar, bevorzugt 90 bis 120 mbar betrieben. Der Verweilzeittank 11 muss derart ausgeführt sein, dass eine mittlere hydrodynamische Verweilzeit des Kreislaufstroms 8 von mindestens 2 Stunden, bevorzugt mindestens 4 Stunden eingestellt wird, um eine effektive und möglichst vollständige Spaltung von BPA und Nebenprodukten zu erreichen. Der Verweilzeitreaktor kann hierbei vollständig gefüllt im Auf- oder Abstrom oder standgeregelt betrieben werden.

Die Isomerisierung und die damit verbundene Spaltung im Verweilzeitbehälter 11 in Schritt d) wird in Anwesenheit eines sauren Katalysators durchgeführt. Als saurer Katalysator (Strom 12) für die Spaltung in Schritt d) kann prinzipiell eine Vielzahl stark saurer schwer- oder nichtflüchtiger Brönstedsäuren zum Einsatz kommen, so unter anderem Phosphorsäure oder deren höhere Kondensate, Schwefelsäure, Alkansulfonsäuren mit >4 Kohlenstoffatomen in der Alkankette, aromatische Sulfonsäuren, Arylalkan-Sulfonsäuren oder Phosphoniumsäuren. Neben diesen homogen eingesetzten Säuren ist auch der Einsatz von heterogenen Spaltungskatalysatoren wie beispielsweise stark saurem Aluminiumoxid, geträgerten Lewissäuren, sauren Zeolithen oder anderen Tonerden oder Polystyrrolsulfonsäuren möglich. In diesem Fall entfällt die Dosiereinheit 13 und der heterogene Katalysator wird durch eine geeignete Rückhaltekonstruktion in den Verweilzeitbehälter 11 eingebracht und bei Bedarf ausgewechselt. In einer bevorzugten Ausführungsform der Erfindung erfolgt die Isomerisierung und Spaltung in Schritt d) in Gegenwart von Schwefelsäure, Phosphorsäure oder p-Toluonsulfonsäure, besonders bevorzugt mit Schwefelsäure. Die Dosierung kann hierbei wie in Figur 1 dargestellt als Strom 12 über die Dosiereinheit 13 in den Kreislaufstrom des Sumpfprodukts der Destillationskolonne 2 erfolgen. Die Dosierung kann aber ebenso in den Eingangsstrom 8 in den Verweilzeitbehälter 11 hinein oder in den Ausgangsstrom 14 aus dem Verweilzeitbehälters 11 heraus erfolgen. Schließlich kann die Dosierung auch in den Teilstrom 1 in die Destillationskolonne 2 erfolgen. Bevorzugt ist die Dosierung in den Kreislaufstrom des Sumpfprodukts der Destillationskolonne 2 der Kolonne.

Die bevorzugte Konzentration des sauren Spaltungskatalysators richtet sich nach der Menge der spaltbaren Nebenprodukte im Eingangsstrom sowie die Art der verwendeten Säure und kann in einfachen Versuchen leicht ermittelt werden. Für die bevorzugte Ausführungsform mit Schwefelsäure ergibt sich die dosierte Säuremenge wie folgt:

$$M \text{ (Schwefelsäure)} = c * (1 - X) * M (1)$$

wobei

M        (Schwefelsäure) den Massenstrom an Schwefelsäure in kg/h, und
c        den Konzentrationsfaktor, und
X        den Massenanteil an Phenol im Teilstrom 1, und
M (1)    den Massenstrom des Teilstroms 1 in kg/h bedeuten.

Hierbei liegt c für eine effektive Spaltung zwischen 0,001 % und 2 %, bevorzugt zwischen 0,005 und 1 % besonders bevorzugt zwischen 0,01 und 0,2 %. Für andere Säuren gelten analoge Gleichungen mit 0,001 % < c < 5 %.

Die Temperatur im Verweilzeitbehälter 11 muss für eine effektive Isomerisierung und Spaltung >190 °C, bevorzugt > 200 °C sein. Um den Einsatz von zusätzlichen Apparaten zu beschränken, erfolgt der Wärmeeintrag in einer bevorzugten Ausführungsform direkt durch den Verdampfer 7 der Destillationskolonne 2. Das bedeutet, dass der Eingangsstrom 8 in den Verweilzeitbehälter 11 hinein nicht durch einen separaten Wärmetauscher aufgeheizt wird und der Sumpf der Kolonne 2 und der Verweilzeitbehälter 11 auf der selben Temperatur betrieben werden.

Um eine effiziente Abtrennung des Phenols aus dem Ausgangsstrom 14 aus dem Verweilzeitbehälter 11 heraus zu gewährleisten ist es nötig, dass der Umwälzstrom über den Verweilzeitbehälter gegenüber dem Teilstrom 1 nicht zu gering ist. Ansonsten erfolgt insgesamt eine nur eine unvollständige Spaltung der spaltbaren Nebenprodukte und eine Ausspeisung noch spaltbaren Materials über den ausgespeisten Teilstrom 9, was mit einem Materialverlust verbunden ist. Daher beträgt der in den Verweilzeitbehälter 11 eingetragene Massenstrom > 30 %, bevorzugt > 80%, ganz besonders bevorzugt > 100% des Massenstroms des aus dem Prozess zur Herstellung von Bisphenol A stammenden Teilstroms 1.

Um die säurekatalysierte Spaltung erfolgreich ablaufen zu lassen ist es darüber hinaus erforderlich die hydrodynamische Verweilzeit im Verweilzeitbehälter 11 mindestens 2 Stunden, bevorzugt mindestens 4 Stunden zu wählen.

**Beispiele**

Die folgenden Beispiele 1 bis 7 werden in einer Versuchsanlage gemäß Figur 1 durchgeführt.

Eine technische Anlage zur Herstellung von Bisphenol A liefert nach Reaktion und Abtrennung von BPA einen ausgespeisten Teilstrom 1, welcher der destillativen Aufarbeitung in der Vakuum-Destillationskolonne 2 zugeführt wird.

Der Teilstrom 1 wird mit einem Massenstrom M(1) der Destillationskolonne 2 zugeführt und enthält Phenol in einem Massenanteil X sowie andere Komponenten in einem Massenanteil (1-X), die im wesentlichen BPA und dessen Isomere sowie verschiedene verzweigte und höhere Kondensationsprodukte von Aceton und Phenol enthalten wie beispielsweise Hydroxyphenyl-substituierte Indane, Chromane, Trisphenole und ähnliche spaltbare Produkte. Die Zusammensetzung (ohne Phenolanteil) beträgt hierbei in allen Beispielen und Vergleichsbeispielen: p,p-BPA 35-40 Gew.-%, o,p-BPA 9 bis 12 Gew.-%, hydroxyphenylsubstituierte Indane 15 bis 19 Gew.-%, hydroxyphenylsubstituierte Chromane 18 bis 22 Gew.-%, Trisphenol 3 bis 5 Gew.-%, sonstige Bestandteile 9 bis 12 Gew.-%. Dies entspricht einer typischen Zusammensetzung eines Ausspeisestroms aus einer Bisphenol-A-Produktionsanlage.

In Tabelle 1 ist die Reinheit C(5) sowie der Massenstrom M(5) des erhaltenen Phenolstroms 5 am Kopf der Kolonne für die den Beispielen 1 bis 7 zugrunde liegenden verschiedenen Bedingungen gezeigt. Die eingesetzte Kolonne 2 weist 20 theoretische Böden auf und wurde mit einem Rücklaufverhältnis von ca. 0,6 und mit den Drücken betrieben, die sich bei Einstellen der Temperatur T(11) am Boden der Kolonne 2 im thermodynamischen Gleichgewicht einstellen. Die Temperatur im Verweilzeitbehälters 11 gleicht dabei der Temperatur am Boden der Kolonne 2. Die angegebenen Werte beziehen sich auf Gleichgewichtsbedingungen, die sich bei kontinuierlichem Betrieb der Kolonne nach wenigen Stunden einstellen.

Für eine optimale Rückgewinnung von Phenol aus dem Teilstrom 1 ist es wünschenswert, den Massenstrom M(5) und die Reinheit C(5) des abgetrennten Phenolstroms 5 zu maximieren, um eine möglichst große Menge an hochreinem Phenol zu gewinnen. Gleichzeitig gilt es, den Massenstrom M(9) an nicht verwertbaren Restharz 9 zu minimieren. In den erfindungsgemäßen Beispielen 1 und 2 wird durch die Spaltung der im Teilstrom 1 enthaltenen spaltbaren Bestandteile der Massenstrom M(5) des abgetrennten Phenolstroms 5 erhöht, so dass sich bezogen auf den Massenanteil X an Phenol im Teilstrom 1 eine Phenolausbeute $Y = M(5) / (X * M(1))$ von > 1,20 ergibt und gleichzeitig ein Phenolstrom 5 hoher Reinheit C(5) gewonnen wird und der Massenstrom M(9) an ausgespeistem Restharz 9 minimiert wird.

In den Beispielen 1 bis 3 und 5 bis 7 wird die Spaltung im Verweilzeitbehälter 11 durch Zugabe von Schwefelsäure unterstützt. Die eingesetzten Mengen an Schwefelsäure M(Schwefelsäure) ergeben sich dabei aus der folgenden Gleichung: $M(Schwefelsäure) = c * (1 - X) * M(1)$.

Im Vergleichs-Beispiel 3 wird die Temperatur T(11) im Verweilzeitbehälter 11 auf 180°C erniedrigt. Dadurch sinkt die Phenolausbeute auf 1,03, d. h. es findet keine effektive Spaltung mehr statt.

Vergleichs-Beispiel 4 zeigt den Einfluss der Schwefelsäurekonzentration. Ohne Einspeisung zusätzlicher Säure (c = 0 in Beispiel 4 bedeutet M(Schwefelsäure) = 0) erfolgt keine effektive Spaltung, die Phenolausbeute sinkt daher auf 0,97.

Vergleichs-Beispiel 5 zeigt den Effekt des Massenstroms des durch den Verweilzeitbehälters 11 geführten Stroms an Sumpfprodukt. Nur bei ausreichendem Massenstrom M(8) des durch den Verweilzeitbehälter 11 und in zurück in die Kolonne 2 geführten Sumpfprodukts erfolgt effektive Spaltung und Rückgewinnung von Phenol. Für $M(8) < 0,3 * M(1)$ sinkt die Phenolausbeute daher auf 1,05.

Vergleichs-Beispiel 6 wird unter ansonsten identischem Aufbau mit einer Kolonne geringerer Trennleistung (Anzahl der theoretischen Böden = 1) durchgeführt. Hierbei wird zwar eine hohe Phenolausbeute Y = 1,27 erreicht, jedoch am Kopf der Kolonne nur eine mäßige Phenolreinheit (C 5) = 96,2 Gew.-%) erzielt.

Vergleichsbeispiel 7 wird mit der bereits in den Beispielen 1 bis 5 Kolonne 2 durchgeführt, jedoch wird der Verweilzeitbehälter 11 umfahren, so dass keine Verweilzeit zur Spaltung zur Verfügung steht. Als Ergebnis wird trotz Schwefelsäuredosierung keine hohe Phenolausbeute Y im Phenolstrom 5 realisiert.

Die Beispiele und Vergleichsbeispiele zeigen, dass in dem erfindungsgemäßen Verfahren unter Einsatz einer Destillationskolonne 2 und eines Verweilzeitbehälters 11 bei Dosierung eines sauren Spaltungskatalysators Phenol hoher Reinheit in erhöhter Ausbeute am Kopf der Kolonne gewonnen und gleichzeitig die Menge an Restharz minimiert werden kann.

**Tabelle 1**

| | M(1) [t/h] | X [-] | c [%] | T(11) [°C] | C(5) [Gew.-%] | M(5) [t/h] | Y [-] | M(9) [t/h] | M(8) [t/h] | $\tau(11)$ [h] |
|---|---|---|---|---|---|---|---|---|---|---|
| Bsp. 1 [1)2)] | 2,75 | 0,65 | 0,06 | 205 | 99,88 | 2,19 | 1,22 | 0,56 | 3,10 | 19,4 |
| Bsp. 2 [1)2)] | 2,40 | 0,50 | 0,06 | 210 | 99,91 | 1,72 | 1,43 | 0,68 | 2,95 | 20,3 |
| Vergl.-Bsp. 3 [1)2)] | 2,65 | 0,62 | 0,06 | 180 | 99,75 | 1,69 | 1,03 | 0,96 | 2,80 | 21,4 |
| Vergl.-Bsp. 4 [1)2)] | 2,80 | 0,59 | 0,0 | 205 | 99,87 | 1,60 | 0,97 | 1,20 | 3,05 | 19,7 |
| Vergl.-Bsp. 5 [1)2)] | 2,75 | 0,63 | 0,05 | 200 | 99,89 | 1,82 | 1,05 | 0,93 | 0,5 | 120,0 |

(fortgesetzt)

| | M(1) [t/h] | X [-] | c [%] | T(11) [°C] | C(5) [Gew.-%] | M(5) [t/h] | Y [-] | M(9) [t/h] | M(8) [t/h] | τ(11) [h] |
|---|---|---|---|---|---|---|---|---|---|---|
| Vergl.-Bsp. 6 [3)2)] | 2,90 | 0,62 | 0,06 | 205 | 96,25 | 2,28 | 1,27 | 0,62 | 3,2 | 18,8 |
| Vergl.-Bsp. 7 [1)4)] | 2,90 | 0,58 | 0,05 | 205 | 99,58 | 1,70 | 1,01 | 1,20 | 3,2 | 0 |

mit M(1) = Massenstrom des Teilstroms 1, X = Phenolanteil in Teilstrom 1, C = Konzentrationsfaktor für Schwefelsäurezugabe M($H_2SO_4$) = c * (1 - X) *M(1); es wurde 96 %ige Schwefelsäure dosiert, T(11) = Temperatur im Verweilzeitbehälter 11, C(5) = Gehalt an Phenol im Phenolstrom 5, M(5) = Massenstrom des Phenolstroms 5, Y = Phenolausbeute bezogen auf den Gehalt an Phenol im Teilstrom 1: Y = M(5) / (X * M(1)), M(9) = Massenstrom an ausgeschleustem Restharz 9 zur Entsorgung, M(8) = Massenstrom an Sumpfprodukt durch den Verweilzeitbehälter 11, τ(11) = hydrodynamische Verweilzeit im Verweilzeitbehälter (11).

Fußnoten:

1) eine Vakuumkolonne mit 20 theoretischen Böden wird eingesetzt 2) ein Verweilzeitbehälter mit einem Volumen von 60 m$^3$ wird eingesetzt

3) eine Vakuumkolonne mit 1 theoretischen Boden wird eingesetzt 4) der Verweilzeitbehälter 11 wird umfahren

**Patentansprüche**

1. Verfahren zur kontinuierlichen Abtrennung von Phenol aus einem bei der Herstellung von Bisphenol A erzeugten Teilstrom enthaltend 40 bis 90 Gew.-% Phenol, 5 bis 40 Gew.-% Bisphenol A sowie 5 bis 40 Gew.-% Nebenkomponenten, die bei der Umsetzung von Phenol und Aceton zu Bisphenol A anfallen, bei dem man

   a) den Teilstrom einer Destillationskolonne enthaltend mindestens 5 theoretische Trennstufen zuführt, und

   b) in der Destillationskolonne Phenol über Kopf abdestilliert, und

   c) einen ersten Teil des Sumpfprodukts aus dem Prozess ausschleust, und

   d) einen zweiten Teil des Sumpfprodukts kontinuierlich in einen Verweilzeitbehälter überführt, in dem man die in dem Sumpfprodukt enthaltenen Nebenkomponenten bei Temperaturen von > 190°C und einer hydrodynamischen Verweilzeit von mindestens 120 min in Gegenwart eines sauren Katalysators zumindest teilweise isomerisiert, und anschließend zurück in die Destillationskolonne führt, wobei der Massenstrom des in den Verweilzeitbehälter geführten Teil des Sumpfprodukts mehr als 30% des Massenstroms des in Schritt a) in die Destillationskolonne eingebrachten Teilstroms beträgt.

2. Verfahren nach Anspruch 1, bei dem man als sauren Katalysator in Schritt d) Schwefelsäure einsetzt.

3. Verfahren nach einem der Ansprüche 1 oder 2, bei dem man die Isomerisierung in Schritt d) bei Temperaturen von > 200°C durchführt.

4. Verfahren nach einem der Ansprüche 1 bis 3, bei dem man die Isomerisierung in Schritt d) mit einer hydrodynamischen Verweilzeit von mindestens 4 Stunden durchführt.

5. Verfahren nach einem der Ansprüche 1 bis 4, bei dem man in Schritt b) Phenol einer Reinheit von > 99,8 Gew.-% erhält.

**Claims**

1. Method of continuously separating phenol from a substream produced in the production of bisphenol A and comprising from 40 to 90 wt% of phenol, from 5 to 40 wt% of bisphenol A and from 5 to 40 wt% of secondary components generated in the reaction of phenol and acetone to afford bisphenol A,
   wherein

   a) the substream is supplied to a distillation column comprising at least 5 theoretical plates, and
   b) phenol is distilled off overhead in the distillation column, and
   c) a first portion of the bottoms product is discharged from the process, and
   d) a second portion of the bottoms product is continuously transferred into a delay vessel, in which the secondary components present in the bottoms product are at least partially isomerized in the presence of an acidic catalyst at temperatures of > 190°C with a hydrodynamic residence time of at least 120 min, and subsequently recycled into the distillation column, wherein the mass flow rate of the portion of the bottoms product passed into the delay vessel is greater than 30% of the mass flow rate of the substream introduced into the distillation column in step a).

2. Method according to Claim 1, wherein the acidic catalyst employed in step d) is sulphuric acid.

3. Method according to either of Claims 1 and 2, wherein the isomerization in step d) is carried out at temperatures of > 200°C.

4. Method according to any of Claims 1 to 3, wherein the isomerization in step d) is carried out with a hydrodynamic residence time of at least 4 hours.

5. Method according to any of Claims 1 to 4, wherein step b) affords phenol of > 99.8 wt% purity.


**Revendications**

1. Procédé de séparation continue de phénol à partir d'un courant partiel formé lors de la production de bisphénol A, contenant 40 à 90 % en poids de phénol, 5 à 40 % en poids de bisphénol A et 5 à 40 % en poids de composants secondaires qui se forment lors de la transformation de phénol et d'acétone en bisphénol A, selon lequel

   a) le courant partiel est introduit dans une colonne de distillation contenant au moins 5 étapes de séparation théoriques, et
   b) le phénol est éliminé par distillation par la tête dans la colonne de distillation, et
   c) une première partie du produit de fond est évacuée du procédé, et
   d) une deuxième partie du produit de fond est transférée en continu dans un contenant à temps de séjour, dans lequel les composants secondaires contenus dans le produit de fond sont au moins partiellement isomérisés à des températures > 190 °C et pendant un temps de séjour hydrodynamique d'au moins 120 minutes en présence d'un catalyseur acide, puis réintroduits dans la colonne de distillation, le courant massique de la partie du produit de fond introduite dans le contenant à temps de séjour étant de plus de 30 % du courant massique du courant partiel introduit dans la colonne de distillation à l'étape a).

2. Procédé selon la revendication 1, selon lequel de l'acide sulfurique est utilisé en tant que catalyseur acide à l'étape d).

3. Procédé selon l'une quelconque des revendications 1 ou 2, selon lequel l'isomérisation à l'étape d) est réalisée à des températures > 200 °C.

4. Procédé selon l'une quelconque des revendications 1 à 3, selon lequel l'isomérisation à l'étape d) est réalisée pendant un temps de séjour hydrodynamique d'au moins 4 heures.

5. Procédé selon l'une quelconque des revendications 1 à 4, selon lequel le phénol est obtenu en une pureté > 99,8 % en poids à l'étape b).

Figure 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 876319 A
- EP 680913 A
- EP 812815 A2